# EUROPÄISCHE PATENTANMELDUNG

(11) **EP 1 788 340 A1**
(43) Veröffentlichungstag der Anmeldung: **23.05.2007**
(21) Anmeldenummer: 06077054.2
(22) Anmeldetag: 15.11.2006
(51) Int. Cl.: F28F 19/02

(54) **Hygieneheizkörper**

(30) Priorität: 21.11.2005 DE 202005018502 U
(71) Anmelder: KERMI GmbH, 94447 Plattling (DE)
(72) Erfinder: Guido, John, 84166 Adelkofen/Deutenkofen (DE)
(74) Vertreter: Bressel und Kollegen

(57) **Zusammenfassung**

Die Erfindung betrifft einen Heizkörper, insbesondere Raumheizkörper mit verbesserten hygienischen Oberflächeneigenschaften, wobei der Heizkörper eine Oberflächenbeschichtung aus einem Pulver- Einbrennlack aufweist, dadurch gekennzeichnet, dass die Oberflächenbeschichtung ein antimikrobielles Additiv aufweist

## Beschreibung

Die Erfindung betrifft einen Heizkörper, der besonderen hygienischen Anforderungen genügt, sowie einen Pulver-Einbrennlack zum Erhalt einer Oberflächenbeschichtung für Heizkörper mit antimikrobieller Wirkung.
Heizkörper dieser Art sind insbesondere für den Einsatz in Einrichtungen mit hohen hygienischen Anforderungen wie Krankenhäusem (Operations- und Eingriffräume, Intensivstationen), Laboratorien, Altenheimen, Sanatorien, Kindereinrichtungen und im Pflegebereich geeignet.
Heizkörper für diese Bereiche sind wie alle üblichen Raumheizkörper beispielsweise als Plan- oder Profilheizkörper, Rundrohrheizkörper oder Heizwand ausgebildet. In Räumen mit hohen hygienischen Anforderungen ist eine möglichst geringe Durchmischung der Raumluft erwünscht, deshalb sind Heizkörper mit einem hohen Strahlungsanteil für diesen Einsatz besonders geeignet, da diese eine geringere Luftzirkulation bzw. Strömungsgeschwindigkeiten erzeugen als Heizkörper die mit Konvektoren ausgestattet sind. Die hohen Anforderungen werden bislang dadurch erfüllt, dass diese Heizkörper konstruktiv so ausgebildet sind, dass sie leicht zu reinigen und desinfizieren sind. Die konstruktive Ausbildung alleine reicht aber noch nicht aus, um den besonders hohen hygienischen Anforderungen in den o.g. Bereichen gerecht zu werden. Die Heizköper müssen außerdem möglichst glatte, nicht poröse Oberflächen aufweisen, die eine leichte Reinigung erlauben. Derartige Oberflächen werden dabei durch eine entsprechende Oberflächenbehandlung mittels Pulver - Einbrennlack durch 2-Schicht-Lackierung erreicht. Die Verfahren zum Aufbringen derartiger Lackierungen sind hinlänglich bekannt (z. B. DE-PS 4136 237).

Der vorliegenden Erfindung liegt die Aufgabe zugrunde einen Heizkörper zu entwickeln, der diese hohen Anforderungen noch besser erfüllt. Die Oberflächen sollen dabei so ausgebildet werden, dass diese nicht nur keine Möglichkeiten für die Anhaftung von Staubpartikeln bieten, so dass sowohl Kontaminationen durch partikelgetragene Mikroorganismen, als auch die Bildung von Verschwelungsprodukten minimiert werden. Darüber hinaus sollen die Oberflächen auch eine antimikrobielle Wirkung aufweisen, da immer mehr Erkrankungen und Infektionen durch Antibiotika resistente Bakterien verursacht werden. Vorteilhaft sind die Oberflächen dabei so ausgebildet, dass eine Ansiedlung von Bakterien, Pilzen und sonstigen gesundheitsgefährdenden Keimen verhindert wird, da ein hoher Anteil derartiger Infektionen über den Kontakt mit kontaminierten Oberflächen erfolgt.

Erfindungsgemäß wird diese Aufgabe durch die Merkmale der Ansprüche 1 und 5 erfüllt. Bevorzugte Ausführungen der Erfindung sind in den zugehörigen Ansprüchen enthalten.

Demnach beinhaltet die Erfindung einen Heizkörper, insbesondere Raumheizkörper mit verbesserten hygienischen Oberflächeneigenschaften, wobei der Heizkörper eine Oberflächenbeschichtung aus einem Pulver- Einbrennlack mit antimikrobiellen Additiv aufweist, woraus sich eine antimikrobielle Wirkung ergibt. Die Oberflächenbeschichtung enthält dabei Komponenten oder Verbindungen die permanent und nachhaltig Bakterien, Pilze und andere gesundheitsgefährdende Keime eliminieren. Besonders bevorzugt werden dabei Silberionen eingesetzt, die vorteilhafter Weise in einer Basispolymerschicht der Oberflächenbeschichtung eingebettet sind. Silber ist oligodynamisch und wirkt in seiner ionischen Form aktiv gegen ein breites Spektrum von schädlichen Mikroben wie Bakterien, Pilzen und Algen. Silberionen sind anorganisch und weniger reaktionsempfänglich und stabiler als organische antibakterielle Substanzen. Dabei besteht die Wirkung nur hinsichtlich einfacher Zellstrukturen. Die komplizierteren Zellstrukturen von Pflanzen, Tieren und Menschen werden dabei nicht angegriffen. Vorteilhaft ist dabei auch, dass die eingesetzten Silberionen keine bakteriellen Resistenzen verursachen.
Die Erfindung bezieht sich weiter auf eine Verwendung eines Pulver- Einbrennlacks zum Erhalt einer Oberflächenbeschichtung für Heizkörper mit antimikrobieller Wirkung, wobei der Pulver- Einbrennlack Komponenten oder Verbindungen enthält die permanent und nachhaltig Bakterien, Pilze und andere gesundheitsgefährdende Keime eliminieren. Bevorzugt werden dabei Silberionen eingesetzt.

Zum Nachweis der antibakteriellen Wirkung wurden Musterbleche mittels Pulver - Einbrennlack durch eine an sich bekannte 2-Schicht-Lackierung lackiert. Dabei wurden zwei Ausführungen hergestellt:
- Musterheizkörper mit einer Lackierung mit einem antbakteriellen Additiv. Dabei wurden Silberionen eingesetzt.
- Musterheizkörper mit einer Lackierung ohne Additiv.
   Die Musterheizkörper wurden künstlich gealtert und dabei den Faktoren Wärme, UV-Strahlung und Umgebungsluftfeuchte ausgesetzt. dabei wurden folgende Belastungsarten ausgewählt:
- Wärmealterung: Angelehnt an DIN 55900-2 Punkt 5.2.6 wurden die Muster in einem Ofen 40 Tage bei einer Temperatur von 110°C gelagert.
- UV-Belastung: Die Muster wurden mit künstlichem UV-Licht belastet. Die Prüfdauer entsprach einer Dosis von 1.473 MJ/m² bei 300 - 800 nm (31 Tage bei 550 W/m²).
- Alterung im Kondenswasserklima: Angelehnt an DIN 55900-2 Punkt 5.2.5 wurden die Muster im Prüfklima DIN 50 017-KFW mit 40 Zyklen (1 Zyklus: 8h mit 40±3 °C, annähernd 100% Luftfeuchte mit Betauung ; 16 h Belüftung mit 23±5°C, unter 100 % Luftfeuchte) belastet.

Im Ergebnis der Untersuchungen wurde festgestellt, dass bei den Musterheizkörpem mit einer Lackierung mit einem Silberionen enthaltenden antbakteriellen Additiv im Gegensatz zu den Musterheizkörpem mit einer Lackierung ohne Additiv, keine gesundheitsgefährdende Keime nachweisbar waren.

## Patentansprüche

1. Heizkörper, insbesondere Raumheizkörper mit verbesserten hygienischen Oberflächeneigenschaften, wobei der Heizkörper eine Oberflächenbeschichtung aus einem Pulver- Einbrennlack aufweist, **dadurch gekennzeichnet, dass** die Oberflächenbeschichtung ein antimikrobielles Additiv aufweist.

2. Heizkörper nach Anspruch 1, **dadurch gekennzeichnet, dass** die Oberflächenbeschichtung Komponenten oder Verbindungen enthält die permanent und nachhaltig Bakterien, Pilze und andere gesundheitsgefährdende Keime eliminieren.

3. Heizkörper nach den Ansprüchen 1 und 2, **dadurch gekennzeichnet, dass** die Oberflächenbeschichtung Silberionen enthält.

4. Heizkörper nach den Ansprüchen 1 bis 3, **dadurch gekennzeichnet, dass** die Silberionen in einer Basispolymerschicht der Oberflächenbeschichtung eingebettet sind.

5. Pulver- Einbrennlack für einen Raumheizkörper nach den Ansprüchen 1-4, **dadurch gekennzeichnet, dass** das Pulver ein antimikrobielles Additiv enthält.

6. Pulver- Einbrennlack für einen Raumheizkörper nach Anspruch 5, **dadurch gekennzeichnet, dass** der Pulver- Einbrennlack Komponenten oder Verbindungen enthält die permanent und nachhaltig Bakterien, Pilze und andere gesundheitsgefährdende Keime eliminieren.

7. Pulver- Einbrennlack für einen Raumheizkörper nach Anspruch 5 und 6, **dadurch gekennzeichnet, dass** der Pulver- Einbrennlack Silberionen enthält.
